# EUROPEAN PATENT APPLICATION

(11) **EP 2 305 692 A1**
(43) Date of publication of application: **06.04.2011**
(21) Application number: 09252302.6
(22) Date of filing: 29.09.2009
(51) Int. Cl.: C07H 1/08, G01N 33/58, G01N 33/68, G01N 30/06

(54) **Method for releasing and labelling O-glycans**

(71) Applicant: Academisch Ziekenhuis Leiden Acting Under The Name Leiden University Medical Center, 2300 RC Leiden (NL)
(72) Inventor: Zauner, Gerhild, 2585 ER Den Haag (NL); Wuhrer, Manfred, 2252 BR Voorschoten (NL)
(74) Representative: Pugh, Robert Ian

(57) **Abstract**

The application describes a one-pot process for releasing and labelling 0-glycans from glycoproteins, thereby facilitating analysis via LC-ESl-MS-MS. The process comprises contacting the glycoprotein with an amine, eg. methylamine, dimethylamine, or ammonia, NH₃ in the presence of a labelling agent, especially 1-phenyl-3-methyl-5-pyrazolone (PMP; CAS Reg. No. 89-25-8), 1-naphthyl-3-methyl-5-pyrazolone (NM P) or 1-(4-methoxy)phenyl-3-methyl-5-pyrazolone (PMPMP, CAS Reg. No. 60798-06-3).

## Description

The invention relates to the analysis of O-glycans.

The listing or discussion of a prior-published document or any background in the specification should not necessarily be taken as an acknowledgement that a document or background is part of the state of the art or is common general knowledge.

Protein glycosylation plays an important role in many biological processes such as molecular adhesion, signal transduction and recognition. Analysis of protein glycosylation is an important first step towards establishing the function these glycans play in health and disease (Haslam et al. Curr. Opin. Struct. Biol. 2006, 16, 584-591).

Both N-glycans and O-glycans can be found on cell surfaces and secreted proteins. Glycans serve as ligands for lectins mediating adhesion and cellular signalling. While techniques for N-glycan analysis are becoming routine, analysis of O-glycans still remains a challenge, as there is no enzyme that efficiently releases all O-linked oligosaccharides from the polypeptide backbone (Schulz et al, Anal. Chem. 2002, 74, 6088-6097; and Geyer et al, Biochem. Biophys. Acta 2006, 1764, 1853-1869).

Several options for chemical release of O-glycans from glycoproteins have been reported such as hydrozinolysis, reductive and non-reductive β-elimination (Geyer et al, Biochim. Biophys. Acta., 2006, 1764, 1853-1869, Mechref et al, Chem. Rev., 2002, 102, 321-369, Harvey, Int. J. Mass Spectrom. 2003, 226, 1-35). The most common way to release and analyze O-linked oligosaccharides from glycoproteins is by reductive β-elimination (Geyer et al, Biochim. Biophys. Acta., 2006, 1764, 1853-1869), followed by solid phase extraction of the O-glycans. O-glycan alditols can then be analysed by such methods as MALDI-TOF-MS after permethylation, or by graphitized carbon HPLC-MS. These methods have their disadvantages. For example, MALDI-TOF-MS requires a further derivatization step and fails to detect small O-glycans due to their overlap with the MALDI matrix region. Graphitized carbon HPLC-MS requires a dedicated graphitized carbon LC-MS system, thereby limiting the applicability of this method.

A further problem encountered when analysing O-glycans is a sequential degradation of reducing end monosaccharides units by consecutive β-elimination reactions. This process is often referred to as "peeling" (Merry et al, Anal. Biochem. 2002, 304, 91-99) and can result in low reaction yields.

Another problem with the above-mentioned chemical release methods is the necessity of sample workup steps, which are very time consuming and also demanding in terms of the equipment required.

In summary, there is a need for a rapid, sensitive and comprehensive method for O-glycan release and analysis.

The present invention addresses the foregoing and other deficiencies by providing a process comprising contacting a glycoprotein with an amine in the presence of a labelling agent to produce at least one labelled O-glycan. The invention also provides a method for detecting at least one O-glycan in a sample, the method comprising contacting the sample with an amine in the presence of a labelling agent, and detecting at least one labelled O-glycan.

Surprisingly, the inventors have found that the release of O-glycans from glycoproteins by an amine in the presence of a labelling agent results in efficient labelling in a "one-pot" reaction. This can be followed by simple sample workup and mass spectrometric analysis.

The invention may be carried out on any glycoprotein. Typically, the O-glycans are released from glycoproteins contained within a protein sample of interest. A protein of interest may be present and/or extracted from a sample from a human patient or animal. A non-limiting example of such a protein is mucin.

Any suitable amine may be used in the invention. In one aspect of the invention, the amine is selected from the group consisting of dimethylamine (DMA), methylamine, ammonia and mixtures thereof. Preferably, the amine is DMA. The amine may be added to the reaction neat or in any suitable solvent (e.g. water).

Any suitable labelling agent may be used in the invention. Typically, the labelling agent should be able to react with the reducing end of an O-glycan. In one embodiment, the labelling agent is selected from 1-phenyl-3-methyl-5-pyrazolone (PMP), 1-(2-naphthyl)-3-methyl-5-pyrazolone, 1-(2-naphtyl)-3-methyl-5-pyrazolone (NMP), 1-(4-methoxy)phenyl-3-methyl-5-pyrazolone (PMPMP) and mixtures thereof. PMP currently is a preferred labelling agent. The labelling agents are either commercially available or may be prepared using known methods (see, for example, You et al, Anal. Chim. Acta 2008, 609 (1), 66-75 or Kakehi et al, J. Chromatogr. 1993, 630 (1-1), 141-6, which are incorporated herein by reference).

Without being bound by theory, it is believed that release of the O-glycans in the presence of a labelling agent reduces or prevents "peeling". This in tum results in a higher yield and a more efficient reaction when compared to existing release and labelling methods.

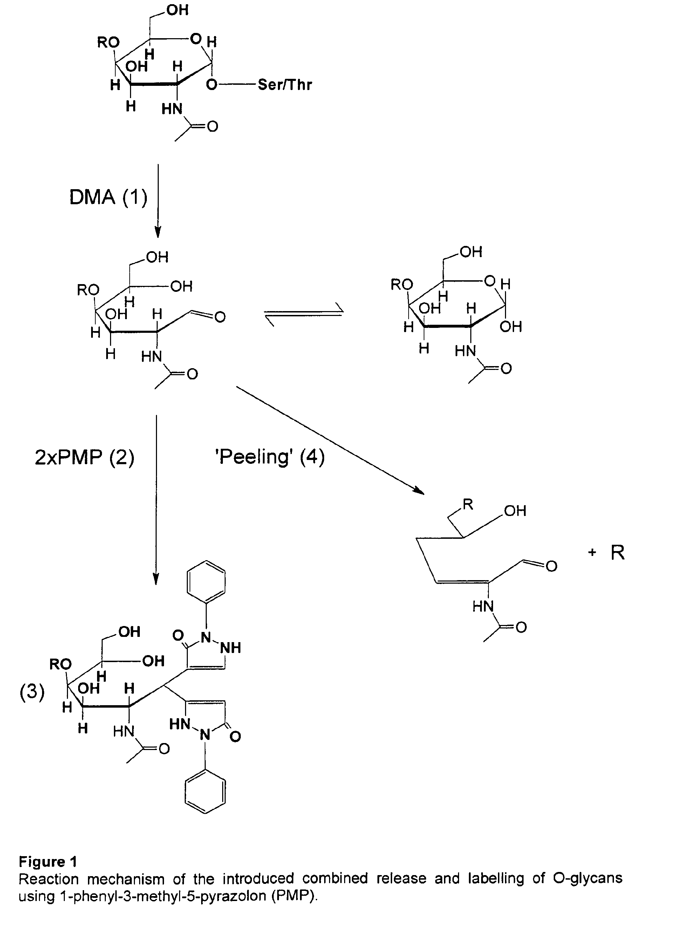

As shown in Figure 1, the release and derivatization occurs in two steps in one pot: a first reaction in which the O-glycan is released from the glycoprotein using an amine, immediately followed by a second reaction in which the reducing end of the released O-glycan reacts with the labelling agent via a Michael type addition, resulting in a 2:1 stoichiometry of labelling agent to O-glycan.

Following labelling, the at least one O-glycan may be purified. Any suitable purification technique may be used. In one embodiment of the invention, the at least one O-glycan can be purified by porous graphitized carbon solid phase extraction (PGC-SPE), cationic exchange resins, liquid-liquid extraction or a mixture of the foregoing. Preferably, PGC-SPE is used, optionally using Carbograph packed as a suspension in water.

The invention allows for the detection of O-glycans from a (glyco)protein sample of interest. Accordingly, the invention provides a method for detecting at least one O-glycan in a sample, the method comprising contacting the sample with an amine in the presence of a labelling agent, and detecting at least one labelled O-glycan.

Any suitable detecting method may be used in the detecting step, such as liquid chromatography, and/or mass spectrometry and/or UV analysis. A preferred method is nano liquid chromatography electrospray ionization tandem mass spectrometry (nano-LC-ESI-MS/MS).

The invention may include a purification step following the contacting the glycoprotein/sample with an amine in the presence of a labelling agent. Typically, such a purification step takes place before any detection step.

Any suitable purification technique may be used. For example, the at least one O-glycan can be purified by porous graphitized carbon solid phase extraction (PGC-SPE), cationic exchange resins, liquid-liquid extraction or a mixture of the foregoing. Preferably, PGC-SPE is used, optionally using Carbograph packed as a suspension in water.

The ease, efficacy, speed and sensitivity of the process and method (compared to the prior art) means that it is applicable to high-throughput analysis. Thus, in one aspect, the invention may be carried out simultaneously on a plurality of samples of glycoprotein(s).

The (one pot) reaction of the at least one glycoprotein with an amine and labelling agent may be carried out at any suitable reaction temperature for any suitable length of time. Typical reactions times are from about 1 minute to about 10 hours, such as from about 5 minutes to about 5 hours (for example from about 10 minutes to about 3 hours).

Typical reaction temperatures are from about 20°C to about 150°C, such as from about 30°C to about 120°C (for example from about 40°C to about 100°C).

The reaction may be carried out in any suitable solvent, such as water, methanol, buffer or a mixture thereof.

Preferably, an excess of labelling agent and amine is used over the glycoprotein. This is thought to result in more efficient O-glycan release and labelling. Typical concentrations are: 50-1000mM (such as 100-500mM, e.g. 250mM) of labelling agent (e.g. PMP) in a suitable solvent (such as an alcohol, e.g. MeOH), 20-60% (such as 30-50%, e.g. 40%) amine (e.g. DMA) in a suitable solvent (e.g. water) and 1-20µg (such as 2-10µg, e.g. 5µg) of glycoprotein of interest.

The process and method of the invention will be explained in more detail with specific reference to the use of DMA and PMP as amine and labelling agent, respectively. The skilled person will appreciate that the invention is not limited to the use of these compounds.

Figure 1 shows the release of O-glycans from glycoproteins using DMA in the presence of 1-phenyl-3-methyl-5-pyrazolon (PMP) which was carried out. The PMP reacted with the reducing end of the freshly released O-glycan structures via a Michael type addition resulting in a 2:1 stoichiometry of PMP per glycan. This is described in more detail below.

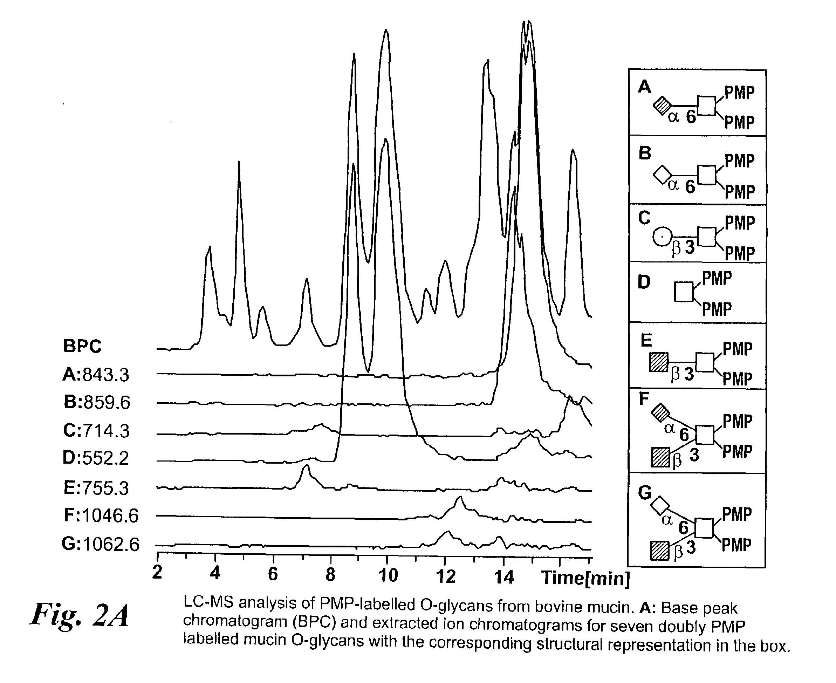

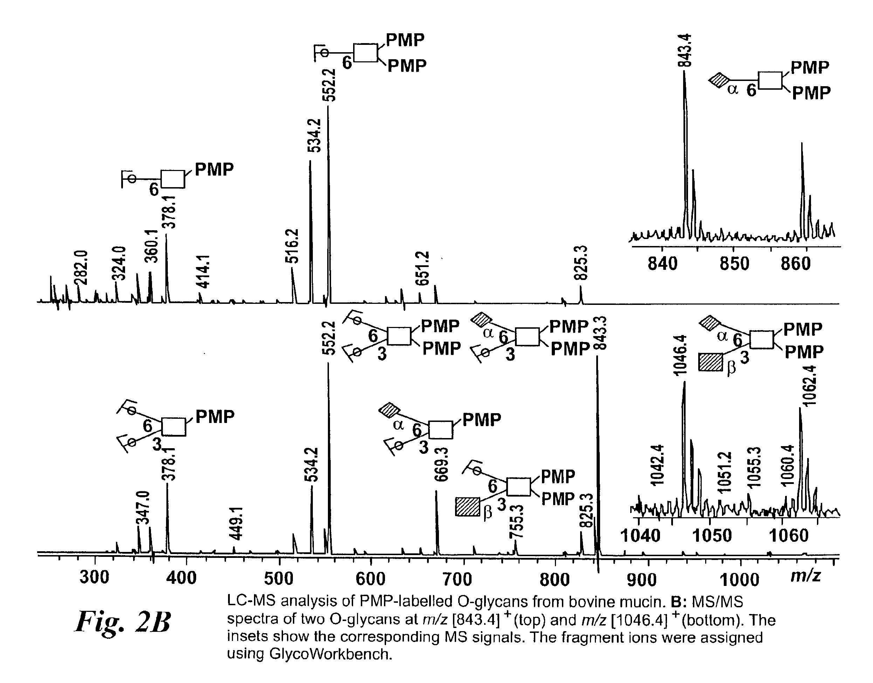

Bovine submaxillary gland mucin was used as a model substance. To determine labelling efficacies, the mucin samples were worked up after O-glycan release and labeling by vacuum centrifugation and graphitized carbon solid phase extraction followed by nano-LC-ESI-MS/MS analysis using a conventional reverse-phase (RP) column, similar to a method for N-glycan analysis (Saba et al, J.Mass Spectrom. 2001, 36 563-574, which is incorporated herein by reference). This procedure resulted in the registration of a set of short-chain O-glycan structures (Figure 2, Table 1), which was in accordance with literature data (Robbe et al, Electrophoresis 2003, 24 611-621, which is incorporated herein by reference).

**Table 1**. Composition and proposed structures of PMP labeled O-glycans from mucin and their measured *m*/*z* values. Light square, N-acetylgalactosamine; circle, galactose; dark square, N-acetylglucosamine; dark diamond, N-acetylneuraminic acid; light diamond, N-glycolylneuraminic acid; N, N-acetylhexosamine; S, sialic acid; H, hexose.

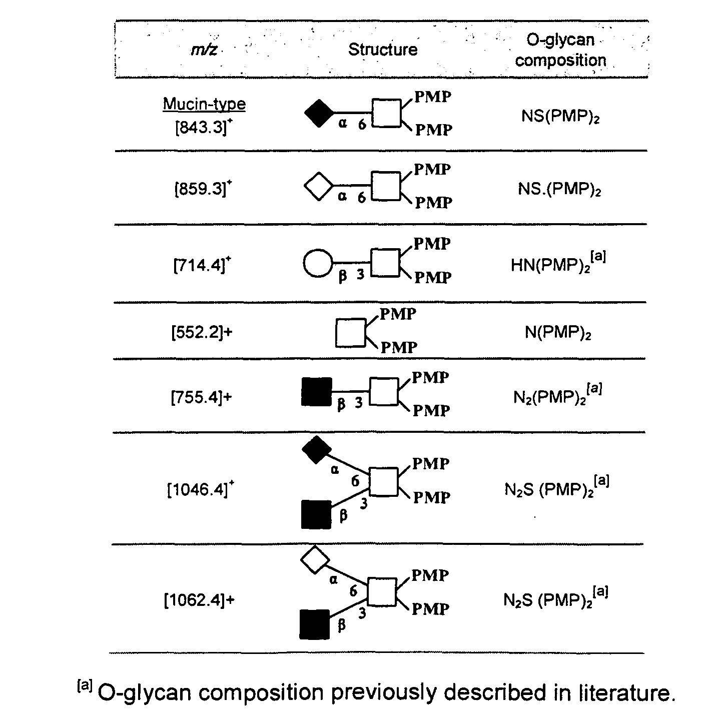

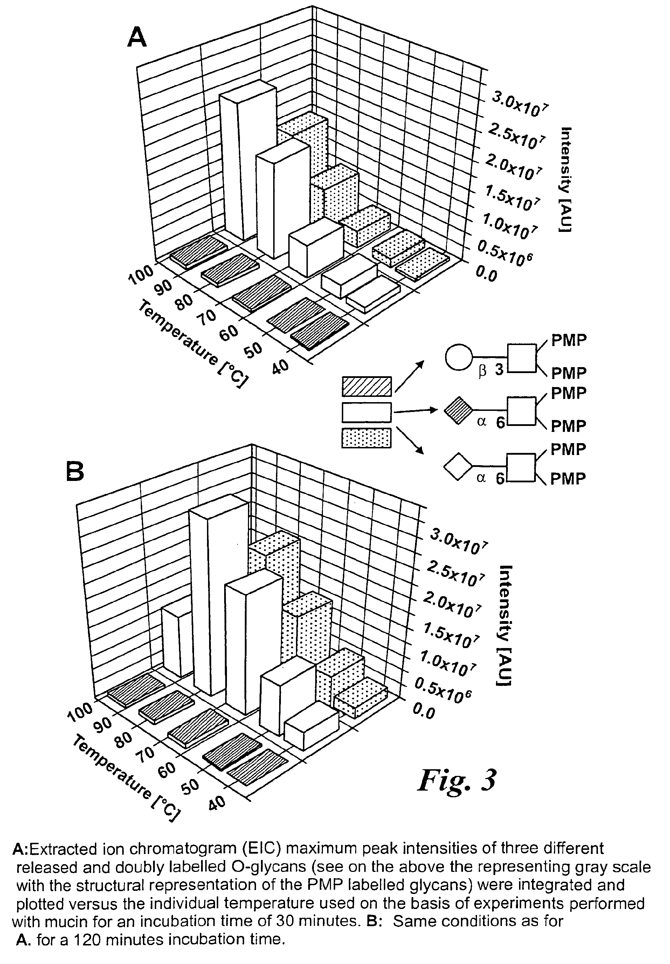

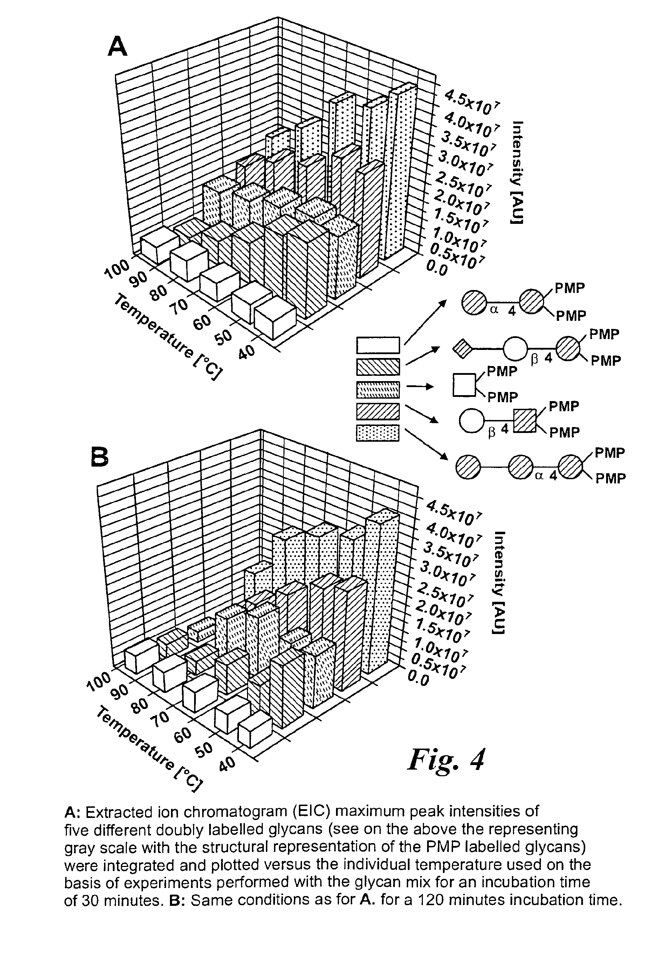

The technique was optimized in terms of reaction conditions such as PMP concentration, temperature, and incubation time to obtain a maximum yield of PMP-labelled O-glycans (Figure 3). Combined β-elimination and PMP labelling in 40% DMA and 250mM PMP for 120 min at 85°C resulted in maximum reaction yield and were chosen as standard conditions. These relatively harsh conditions were necessary for the most efficient glycan release, while in contrast the labelling took place very rapidly under milder conditions (Figure 4). Control experiments were also performed; these are described in more detail later in this specification.

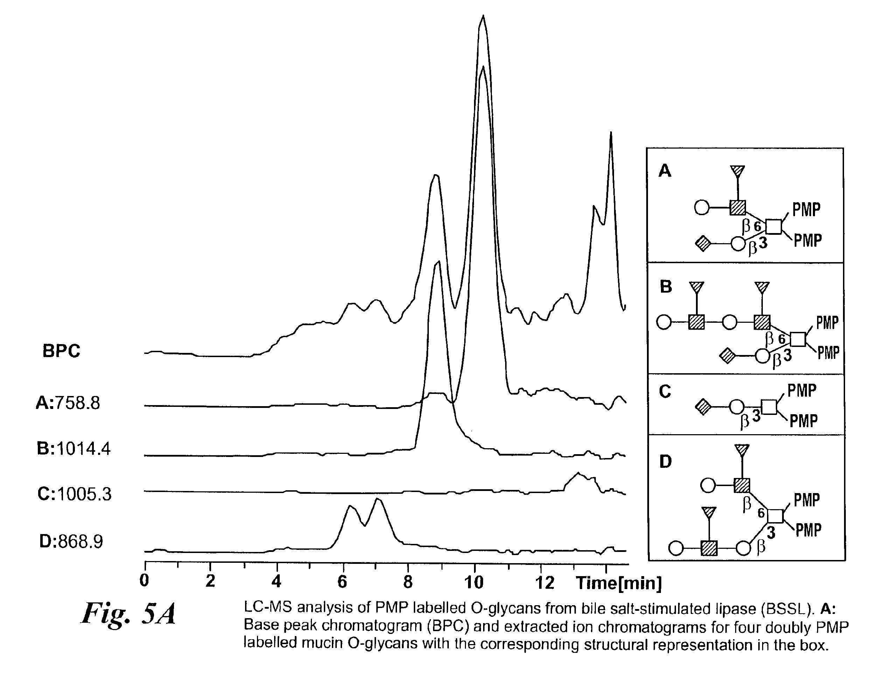

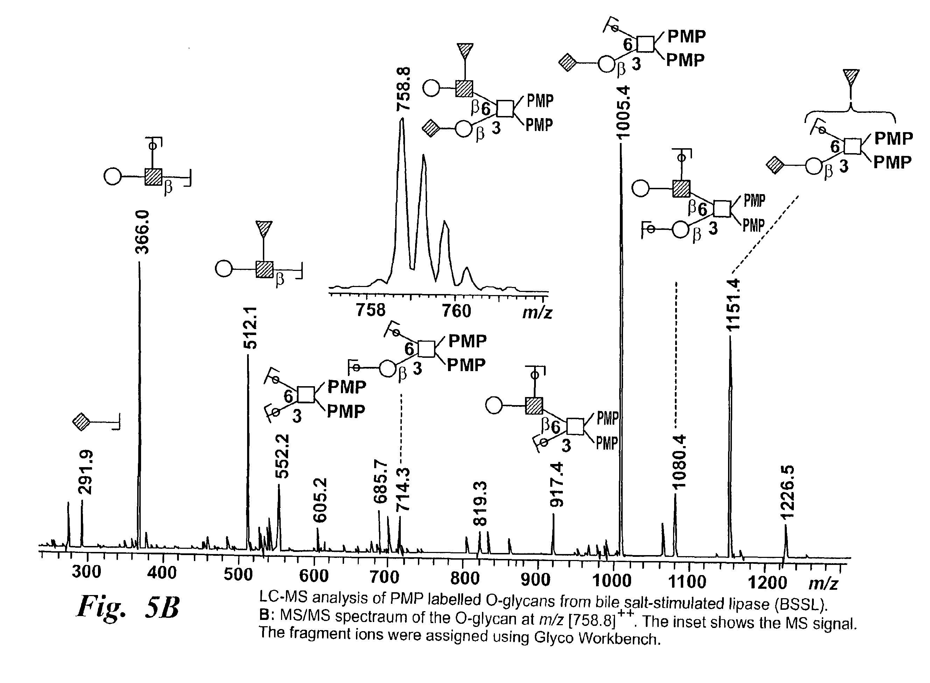

The analysis of the O-glycosylation of bile salt-stimulated lipase (nBSSL) was also carried out (Figure 5). All the PMP labeled O-glycan species detected are listed in Table 2. The detected O-glycans were in accordance with previously published results (Wilson et al, J Proteome.Res. 2008, 7 3687-3696, which is incorporated herein by reference). Additional O-glycan structures could be identified for which a structure was suggested based on the tandem MS data. No peeling products were detected.

**Table 2.** Composition and proposed structures of PMP labeled O-glycans from BSSL and their measured *m*/*z* values. Light square, N-acetylgalactosamine; circle, galactose; dark square, N-acetylglucosamine; triangle, fucose; diamond, N-acetylneuraminic acid. N, N-acetylhexosamine; S, sialic acid; H, hexose; F, deoxyhexose.

| m/z | Structure | O-glycan composition |
|---|---|---|
| BSSL-type [552.2]⁺ | 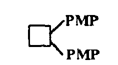 | N(PMP)₂ |
| [714.3]⁺ | 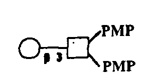 | HN(PMP)₂ |
| [1005.3]⁺ | 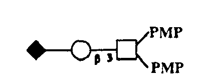 | HNS(PMP)₂^{[b]} |
| [1370.5]⁺ [685.8]⁺⁺ | 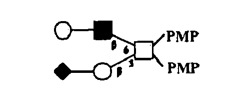 | H₂N₂S(PMP)₂^{[b]} |
| [1225.5]⁺ | 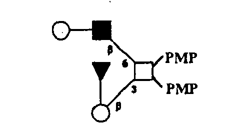 | FH₂N₂(PMP)^{[b]} |
| [1516.6]⁺ [758.8]⁺⁺ | 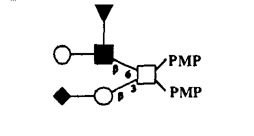 | FH₂N₂S(PMP)₂^{[b]} |
| [795.8]⁺⁺ | 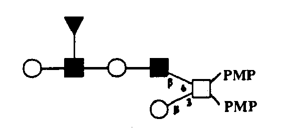 | FH₃N₃(PMP)₂ |
| [868.9]⁺⁺ | 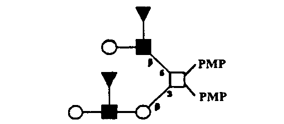 | F₂H₃N₃(PMP)₂ |
| | 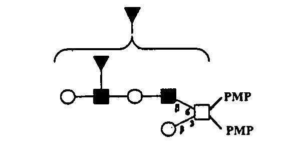 | F₂H₃N₃(PMP)₂ |
| [941.8]⁺⁺ | 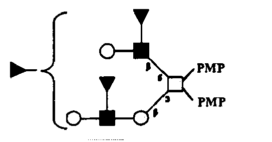 | F₃H₃N₃(PMP)₂ |
| [941.3]⁺⁺ | 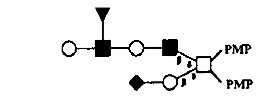 | FH₃N₃S(PMP)₂^{[b]} |
| [1014.4]⁺⁺ | 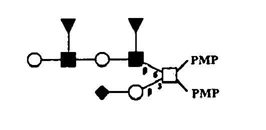 | F₂H₃N₃S(PMP)₂ |
| [1087.4]⁺⁺ | 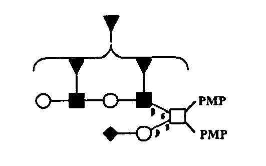 | F₃H₃N₃S(PMP)₂ |
| [978.3]⁺⁺ | 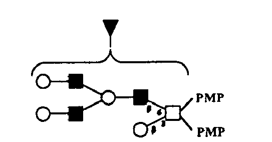 | FH₄N₄(PMP)₂ |
| [1051.4]⁺⁺ | 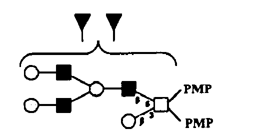 | F₂H₄N₄(PMP)₂ |
| [1124.4]⁺⁺ | 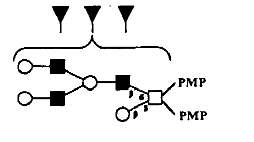 | F₃H₄N₄(PMP)₂^{[b]} |
| [1196,9]⁺⁺ | | F₂H₄N₄S(PMP)₂ |
| [798.3]⁺⁺⁺ | | F₂H₃N₄S(PMP)₂ |

| | | |
|---|---|---|
| ^{[b]} O-glycan composition previously described in literature. For some masses no glycan structure was suggested. | | |

The experimental protocol for the combined release and labelling of O-glycans using DMA and PMP described above is set out below.

### Materials and Reagents

1-phenyl-3-methyl-5-pyrazolon (PMP) was obtained from VWR Prolabo (Amsterdam, The Netherlands). Dimethylamine (DMA) and mucin from bovine submaxillary glands (Type I-S) were obtained from Sigma-Aldrich (Zwijndrecht, The Netherlands). Acetonitrile was purchased from Biosolve (Valkenswaard, The Netherlands). SPE bulk sorbent carbograph was purchased from Alltech (Breda, The Netherlands). Bile salt-stimulated lipase (BSSL) was isolated from human milk and was a kind gift from Dr. Eva Landberg (University of Linköping, Sweden). N',N',N'-triacetylchitotriose, maltotriose, maltotetrose, N-acetyl-D-galactosamine and N-acetyl-lactosamine were obtained from Sigma-Aldrich (Zwijndrecht, The Netherlands). D-(+)-Galactose was obtained from Merck (Darmstadt, Germany).

### Preparation of oligosaccharide mix

A stock solution containing 2.5µg/50µL of each oligosaccharide (N',N',N'-Triacetylchitotriose, Maltotriose, Maltotetrose, N-acetyl-D-galactosamine, N-acetyl-lactosamine, and D(+)-galactose) in water is referred to as glycan mix.

### Combined release and labeling of O-glycans

1 µL of a 5mg/mL stock solution in water of the individual protein of interest (or the glycan mix; see above) was mixed with 2µL of DMA and 3µL of a 0.5M PMP solution in methanol before incubation at 85°C for 120 minutes. After allowing the samples to cool down to room temperature the excess DMA was removed by 5 minutes of vacuum centrifugation at 65°C. Samples were taken up in 150µL of water prior to the following SPE step.

### Sample work-up by PGC-SPE

Oligosaccharide release/labelling samples were purified by porous graphitized carbon (PGC) SPE using Carbograph (20 mg/well) packed as a suspension in water into a 96-well filter plate plate (Orochem OF1100, Orochem, Lombard, IL, USA). The wells were prewashed using a vacuum manifold (Millipore, Billerica, MA; maximum of 170 mbar vacuum) as described by Ruhaak et at (Anal.Chem. 2008, 80 6119-6126, which is incorporated herein by reference), before the samples were loaded to the wells. After two washes with 200µL of water an elutions was performed using 150µL of 50% of acetonitrile in water containing 0.1 % TFA. The eluate were individually collected in 96-well V-bottom deep well plates. The samples were brought to dryness using vacuum centrifugation before re-suspension in 150µL of water for further analysis.

### Nano-LC-ESI-MS/MS

Purified doubly PMP labeled glycans were separated using reverse phase-high-performance liquid chromatography on a PepMap column (3µm; 75µm x 150mm; Dionex, Amsterdam, The Netherlands) using an ultimate 3000 nanoLC sytem (Dionex) equipped with a guard column (5µm; 300µm x 5mm; Dionex). The system was controlled by Chromeleon software.

The system was equilibrated with eluent A (0.1% formic acid in water) at a flow rate of 300nL/min. After injection of the sample (0.2 - 5µl), an immediate step to 26% solvent B (95% ACN) was performed, followed by a linear gradient to 76% solvent B within 30 min and 5 min of isocratic elution. The nano LC system was directly coupled to an Esquire High Capacity Trap (HTCultra) ESI-IT-MS (Bruker) equipped with an online nanospray source operating in a range from 900-1200V in the positive ion mode (nano-bore emitters E523, length 50mm, 30µM i.d.; Proxeon Biosystems, Odense, Denmark). The solvent was evaporated at 165°C with a nitrogen stream of 6L/min. Ions from m/z 100- 2800 were detected. Automatic fragment ion analysis was enabled, resulting in MS/MS spectra of the 10 most abundant peaks.

### Data processing

The LC-ESI-MS/MS data were analysed using Data analysis version 4.0 software (Bruker Daltronics). Peak intensities were determined for doubly PMP labelled species taking into account an averaged background intensity subtraction. Fragment ions were assigned using GlycoWorkbench (Ceroniet al, J Proteome.Res. 2008, 71650-1659).

### Control experiments

In order to check whether DMA is influencing the efficiency of the labeling reaction a glycan mix (see above) was prepared and subjected to the O-glycan release/labeling procedure. Notably all oligosaccharides present in the sample solution could be identified with a double PMP tag in MS/MS mode. Labeling was most efficient at low temperatures (45□C) and short incubation times (30 min). Together with the results obtained for mucin O-glycan release and labeling, this indicated that O-glycan release is the rate-limiting step requiring harsh conditions (incubation for 120 min at 85 °C) whilst the labeling is taking place very rapidly (Figure 3).

The present inventors have shown for the first time a method for O-glycan analysis that combines O-glycan release and labelling in a one-pot reaction. The whole analytical procedure can be completed within several hours, which is much faster than previously described methods such as hydrozinolysis or (non) reductive β-elimination.

The invention may be successfully applied to sub-microgram quantities of glycoprotein. The invention allows for the analysis of short O-glycans such as O-linked GaINAc as well as more extended sialylated O-glycans, and can allow identification of new oligosaccharide structures.

In summary, the present inventors have shown a fast and efficient method for the combined release and labelling of O-glycans, allowing their sensitive MS detection using standard nanoLC-MS equipment.

## Claims

1. A process comprising contacting a glycoprotein with an amine in the presence of a labelling agent to produce at least one labelled O-glycan.

2. A method for detecting at least one O-glycan in a sample, the method comprising contacting the sample with an amine in the presence of a labelling agent, and detecting at least one labelled O-glycan.

3. A process or method according to claim 1 or 2, wherein the labelling agent is selected from 1-phenyl-3-methyl-5-pyrazolone (PMP), 1-(2-naphthyl)-3-methyl-5-pyrazolone, 1-(2-naphtyl)-3-methyl-5-pyrazolone (NMP), 1-(4-methoxy)phenyl-3-methyl-5-pyrazolone (PMPMP) and mixtures thereof.

4. A process or method according to claim 3, wherein the labelling agent is PMP.

5. A process or method according to any of the preceding claims wherein the amine is selected from dimethylamine (DMA), methylamine, ammonia and mixtures thereof.

6. A process or method according to claim 5 wherein the amine is DMA.

7. A process or method according to any of the preceding claims further comprising a step for purifying the at least one labelled O-glycan.

8. A process or method according to claim 7 wherein the purification step comprises porous graphitized carbon solid phase extraction (PGC-SPE), cationic exchange resins, liquid-liquid extraction or a mixture thereof.

9. A process or method according to claim 8 wherein the purification step comprises PGC-SPE.

10. A method according to any of claims 2 to 9 wherein the detecting step comprises nano-LC-ESI-MS/MS analysis.

11. A process or method according to any of the preceding claims, which is carried out simultaneously on a plurality of samples.

12. Any novel process generally as herein described, optionally with reference to the examples.

13. Any novel method as herein described, optionally with reference to the examples.
